# EUROPEAN PATENT APPLICATION

(11) **EP 4 659 793 A2**
(43) Date of publication of application: **10.12.2025**
(21) Application number: 25211636.3
(22) Date of filing: 28.08.2019
(51) Int. Cl.: A61M 25/09

(54) **GUIDE WIRE**

(62) Divisional of application: 19943616.3
(71) Applicant: ASAHI INTECC CO., LTD., Seto-shi, Aichi 4890071 (JP)
(72) Inventor: YOSHIDA, Kenji, Seto-shi, Aichi, 489-0071 (JP); USHIDA, Keisuke, Seto-shi, Aichi, 489-0071 (JP); KASHIWAI, Masahiro, Seto-Shi, Aichi, 489-0071 (JP)
(74) Representative: Prinz & Partner mbB

(57) **Abstract**

A guide wire includes a core shaft formed of superelastic metal, where the core shaft has a tapered portion having a diameter reducing from its own proximal end side toward the distal end side, an intermediate portion having an approximately cylindrical shape adjacent to the proximal end side of the tapered portion, and a proximal end portion adjacent to the proximal end side of the intermediate portion and having a diameter more increased than the intermediate portion; a coil body covering at least a part of the intermediate portion and the tapered portion of the core shaft, the coil body having an outer diameter of 0.36 mm or less; and a distal tip disposed at the distal end of the coil body and forming the distal end of the guide wire, the diameter of the intermediate portion being 0.22 mm or more to 0.24 mm or less.

## Description

### TECHNICAL FIELD

The disclosed embodiments relate to a guide wire.

### BACKGROUND ART

There have been known guide wires used in insertion of a catheter and the like into a blood vessel. Among such guide wires, several configurations have been disclosed with a superelastic shaft formed of nickel-titanium (Ni-Ti) alloy in the vicinity of the distal end (see, e.g., Patent Literatures 1-2).

### CITATION LIST

### Patent Literature

Patent Literature 1: JP2013-544575W
Patent Literature 2: JP2005-46603A

### SUMMARY OF INVENTION

### TECHNICAL PROBLEM

In guide wires having a shaft formed of nickel-titanium alloy, there has been need for a technology capable of suppressing delay of torque response.

Note that such problem is not limited to the blood vascular system, but is common in guide wires to be inserted into each of the organs in the human body such as the lymph gland system, biliary system, urinary system, respiratory tract system, digestive organ system, secretory gland, or genital organs. Moreover, such problem is not limited to a guide wire having a shaft formed of nickel-titanium alloy, but is common in guide wires having a shaft formed of superelastic metal.

The disclosed embodiments were made to solve the problem described above, and have an object to provide a technology of suppressing delay of torque response in a guide wire.

### SOLUTION TO PROBLEM

The disclosed embodiments have been made to solve at least a part of the problem described above, and can be implemented as the following aspects.
(1) One aspect of the disclosed embodiments provides a guide wire. This guide wire includes a core shaft formed of superelastic metal, wherein the core shaft has a tapered portion having a diameter reducing from its own proximal end side toward the distal end side, an intermediate portion having an approximately cylindrical shape adjacent to the proximal end side of the tapered portion, and a proximal end portion adjacent to the proximal end side of the intermediate portion and having a larger diameter than the intermediate portion; a coil body covering at least a part of the intermediate portion and the tapered portion of the core shaft, the coil body having an outer diameter of 0.36 mm or less; and a distal tip disposed at the distal end of the coil body and forming the distal end of the guide wire, wherein the diameter of the intermediate portion of the core shaft is 0.22 mm or more to 0.24 mm or less.
   This configuration provides an intermediate portion of a core shaft with a relatively thin diameter, 0.22 mm or more to 0.24 mm or less, and thus allows suppression of delay of torque response. In other words, a guide wire with better rotational performance can be provided. Moreover, the guide wire includes a coil body with an outer diameter of 0.36 mm or less that covers at least a part of the intermediate portion and a tapered portion of the core shaft, and thus enables ensuring a proper size of the guide wire even in a thin part of the core shaft. Providing the intermediate portion of the core shaft with a thinner diameter allows the wire diameter of the coil body to be thicker, and thus enables suppressing occurrence of riding up and collapse of the coil body during use of the guide wire.
(2) In the guide wire of the aspect described above, the coil body may have a constant wire diameter. The configuration with the wire diameter of a coil body on the proximal end side smaller than the wire diameter of the coil body on the distal end side is more likely to cause occurrence of riding on, collapse, or the like of the coil body during use of the guide wire, in a part with a smaller wire diameter. By contrast, this configuration is set to have a core shaft with a diameter of the intermediate portion of 0.22 mm or more to 0.24 mm or less, thereby allowing a wired shape of a coil body to have relatively thick, constant size, and thus enabling suppressing occurrence of riding on, collapse, or the like of the coil body during use of the guide wire.
(3) In the guide wire of the aspect described above, the core shaft may be formed of nickel-titanium (Ni-Ti) alloy. Nickel-titanium alloy has superior restorability, durability, and corrosion resistance, and this configuration can provide a guide wire with superior restorability, durability, and corrosion resistance in a core shaft.
(4) In the guide wire of the aspect described above, the proximal end portion of the coil body may be fixed to the intermediate portion of the core shaft. This provides a coil body wound around from a predetermined position of the intermediate portion of the core shaft to the distal end of the guide wire, and thus allows increased flexural rigidity of the intermediate portion and a tapered portion, which are relatively thinner parts in the guide wire.
(5) In the guide wire of the aspect described above, a proximal end core shaft may be further included which is connected to the proximal end side of the core shaft and formed of a material with higher rigidity than that of the core shaft. This provides a proximal end core shaft with high rigidity, and thus allows improved pushdown and delivery.
(6) In the guide wire of the aspect described above, the proximal end core shaft may be formed of stainless steel. Because of superior formability of stainless steel, this configuration allows easy production of a guide wire with high pushdown and delivery properties and superior rotational performance.
(7) In the guide wire of the aspect described above, a wire rod may be further included which is connected to the distal end of the core shaft and formed of a material having more plastic deformability than that of the core shaft, and the distal tip may be connected between the distal end of the coil body and the distal end of the wire rod. This allows the distal end part of a guide wire to be easily shaped.

Note that the disclosed embodiments can be achieved in various aspects, for example, in a form of a core shaft product or the like formed of a plurality of core shafts used in a guide wire.

### BRIEF DESCRIPTION OF DRAWINGS

Fig. 1 is a partial sectional view showing a configuration of a guide wire of a first embodiment.
Fig. 2 shows relation of the diameter of an intermediate portion and rotational performance.
Fig. 3 is an illustration to illustrate an evaluation test for rotational performance.
Fig. 4 shows rotational performance of the guide wire of the embodiment.
Fig. 5 is an illustration to illustrate the evaluation test for rotational performance shown in Fig. 4.
Fig. 6 is a partial sectional view showing a configuration of a guide wire of a second embodiment.
Fig. 7 is a partial sectional view showing a configuration of a guide wire of a third embodiment.
Fig. 8 is a partial sectional view showing a configuration of a guide wire of a fourth embodiment.
Fig. 9 is a partial sectional view showing a configuration of a guide wire of a fifth embodiment.

### EMBODIMENTS OF THE INVENTION

### <First Embodiment>

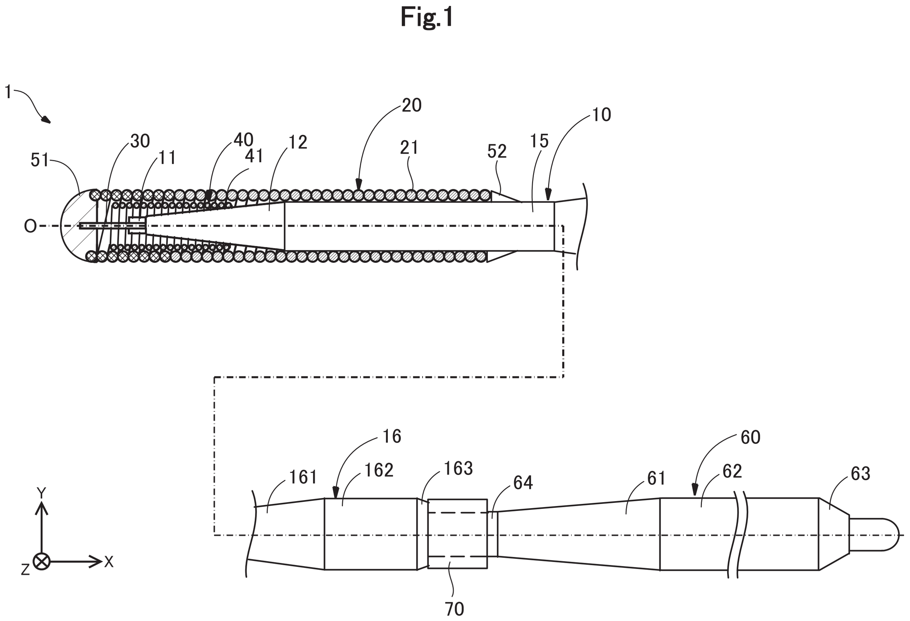

Fig. 1 is a partial sectional view showing a configuration of a guide wire 1 of a first embodiment. The guide wire 1 is a medical appliance used in, e.g., insertion of a catheter into a blood vessel, and includes a core shaft 10, a coil body 20, a wire rod 30, a coated part 40, a distal tip 51, a proximal end fixing part 52, and a proximal end core shaft 60. Fig. 1 indicates an axis passing through the center of the guide wire 1 by an axis O (dash-dot line). In the subsequent examples, all of an axis passing through the center of the core shaft 10 closer to the proximal end than an intermediate portion 15, an axis passing through the center of the coil body 20, and an axis passing through the center of the coated part 40 are identical to the axis O. However, each of the axis passing through the center of the core shaft 10, the axis passing through the center of the coil body 20, and the axis passing through the center of the coated part 40 may be different from the axis O.

Fig. 1 illustrates XYZ-axes orthogonal to each other. The X-axis corresponds to an axis direction of the guide wire 1, the Y-axis corresponds to a height direction of the guide wire 1, and the Z-axis corresponds to a width direction of the guide wire 1. The left side in Fig. 1 (-X-axis direction) is designated as "distal end side" of the guide wire 1 and each component member, and the right side in Fig. 1 (+X-axis direction) is designated as "proximal end side" of the guide wire 1 and each component member. Additionally, in the guide wire 1 and each component member, an end part located at a distal end side is designated as "distal end portion" or simply as "distal end ", and an end part located at a proximal end side is designated as "proximal end portion" or simply as "proximal end". In the embodiment, a distal end side corresponds to "farther side", and a proximal end side corresponds to "nearer side". These points are also common to the drawings showing total configurations subsequent to Fig. 1.

The core shaft 10 is a tapered long-length member having a thicker diameter in the vicinity of the proximal end and a thinner diameter in the vicinity of the distal end. The core shaft 10 is formed of nickel-titanium (Ni-Ti) alloy, which is a superelastic metal. The distal end side of the core shaft 10 is connected to the wire rod 30, and the proximal end side of the core shaft 10 is connected to the proximal end core shaft 60. The core shaft 10 has a small-diameter portion 11, a tapered portion 12, an intermediate portion 15, and a proximal end portion 16, in order from the distal end side to the proximal end side. The length of each part can be determined arbitrarily.

The small-diameter portion 11 of the core shaft 10 is positioned on the distal end side of the core shaft 10. The small-diameter portion 11 is a part having the minimum outer diameter in the core shaft 10, and is formed as an approximately cylindrical shape having a constant outline.

The tapered portion 12 is positioned between the small-diameter portion 11 and the intermediate portion 15. The tapered portion 12 takes an approximately truncated cone shape that has an outer diameter reduced from the proximal end side to the distal end side.

The intermediate portion 15 is adjacent to the proximal end side of the tapered portion 12, and positioned between the tapered portion 12 and the proximal end portion 16. The intermediate portion 15 takes an approximately cylindrical shape that has a constant outer diameter larger than the outer diameter of the small-diameter portion 11. In the embodiment, the diameter of the intermediate portion 15 is 0.237 mm or more to 0.243 mm or less. As described in detail later, the guide wire 1 of the embodiment includes a relatively thin diameter of the intermediate portion 15, and thus allows suppressing delay of torque response.

The proximal end portion 16 is adjacent to the proximal end side of the intermediate portion 15 and has a larger diameter than the intermediate portion 15. The proximal end portion 16 includes a first increased-diameter portion 161, a first large-diameter portion 162, and a first reduced-diameter portion 163. The first increased-diameter portion 161 takes an approximately truncated cone shape that has a diameter of the distal end side identical to the diameter of the intermediate portion 15, and an outer diameter increased from the distal end side to the proximal end side. The first large-diameter portion 162 is a part having the maximum outer diameter in the core shaft 10, and takes an approximately cylindrical shape having a constant outer diameter. The first reduced-diameter portion 163 takes an approximately truncated cone shape that has a diameter of the distal end side identical to the diameter of the first large-diameter portion 162, and an outer diameter reduced from the distal end side to the proximal end.

The outer sides of the small-diameter portion 11, the tapered portion 12, and a part close to the distal end of the intermediate portion 15 are covered with the coil body 20 as described later. By contrast, the proximal end portion 16 is not covered with the coil body 20, and is exposed from the coil body 20.

The coil body 20 takes an approximately cylindrical hollow shape formed by spirally winding a wire 21 onto the core shaft 10 and the wire rod 30. The wire 21 forming the coil body 20 may be a single line composed of one wire, or a strand stranded with a plurality of wires. When the wire 21 is a single line, the coil body 20 is configured as a single coil; and when the wire 21 is a strand, the coil body 20 is configured as a hollow strand coil. Furthermore, a single coil and a hollow strand coil may be combined to configure the coil body 20. The line diameter of the wire 21 and the mean coil diameter in the coil body 20 (the mean diameter of the outer diameter and the inner diameter of the coil body 20) can be determined arbitrarily. The outer diameter of the coil body 20 is 0.36 mm or less, and the line diameter of the wire 21 (also referred to as wire diameter) is constant.

The proximal end portion of the coil body 20 is fixed to the intermediate portion 15 of the core shaft 10 with the proximal end fixing part 52. The proximal end fixing part 52 can be formed of any bonding agent, e.g., metal solder such as silver solder, gold solder, zinc, Sn-Ag alloy, or Au-Sn alloy, or adhesive such as epoxy-based adhesive. The proximal end fixing part 52 can be placed at any position in the intermediate portion 15. It is preferable that the proximal end fixing part 52 be positioned at the proximal end of the intermediate portion 15, because this allows the coil body 20 to largely cover the intermediate portion 15 and provides improved flexural rigidity of the intermediate portion 15, which has a relatively thin diameter.

The wire 21 can be formed of, e.g., stainless alloy such as SUS 304, or SUS 316; superelastic alloy such as Ni Ti alloy; a piano wire; radiolucent alloy such as nickel-chromium-based alloy or cobalt alloy; radiopaque alloy such as gold, platinum, tungsten, or alloy containing these elements (e.g., platinum nickel alloy). Note that the wire 21 may be formed of a known material other than those described above. In the embodiment, the wire 21 is formed of platinum nickel (Pt-Ni) alloy in a portion covering the wire rod 30, and of stainless alloy in a portion closer to the proximal end. This allows the vicinity of the distal end of the guide wire 1 to be shaped easily. Fig. 1 indicates the wire 21 with cross hatching in a portion formed of platinum nickel (Pt-Ni) alloy, and diagonal hatching in a portion formed of stainless alloy.

The wire rod 30 is a long-length member having a constant outer diameter from the proximal end side to the distal end side. The cross-sectional shape of the wire rod 30 is made as an approximately elliptic shape having a major axis and a minor axis. The wire rod 30 is positioned adjacent to the small-diameter portion 11 of the core shaft 10, with the major axis in the Y-axis direction and the minor axis in the Z-axis direction. The wire rod 30 is formed of a material having more plastic deformability than that of the core shaft 10, e.g., stainless alloy such as SUS 302, SUS 304, or SUS 316. The wire rod 30 is formed of a material having more plastic deformability than that of the core shaft 10, and thus allows the distal end part of the guide wire 1 to be shaped more easily relative to a guide wire without the wire rod 30. The wire rod 30 is also referred to as "ribbon". The proximal end side of the wire rod 30 is bonded to the small-diameter portion 11 on the distal end side of the core shaft 10 with a bonding agent. Examples of the bonding agents available include metal solder such as silver solder, gold solder, zinc, Sn-Ag alloy, or Au-Sn alloy, or adhesive such as epoxy-based adhesive. The distal end side of the wire rod 30 is fixed with the distal tip 51 described later.

Note that in the example in Fig. 1, the wire rod 30 is bonded to the core shaft 10, with the position of the proximal end portion of the wire rod 30 matching the position of the proximal end portion of the small-diameter portion 11, in the axis O (X-axis) direction. However, there may be a difference between the position of the proximal end portion of the wire rod 30, and the position of the proximal end portion of the small-diameter portion 11 in the axis O direction. For example, the proximal end portion of the wire rod 30 may be positioned forward in the -X-axis direction relative to the proximal end portion of the small-diameter portion 11. Moreover, the wire rod 30 may be connected to the distal end of the small-diameter portion 11, or inserted in and connected to the small-diameter portion 11.

The coated part 40 is a multiple thread coil having a plurality (e.g., 8) of the wires 41 wound in multiple rows, and is configured to have less plastic deformability than the wire rod 30 and more plastic deformability than the core shaft 10. The coated part 40 can be formed by, e.g., densely stranding a plurality of the wires 41 so as to contact each other on a core bar, then removing remaining stress with a known heat treatment method, and extracting the core bar. The material of the wire 41 may be the same as or different from that of the wire 21. The proximal end side of the coated part 40 is bonded to the tapered portion 12 of the core shaft 10 with any bonding agent similar to the proximal end fixing part 52. The coated part 40 enables reduction of rigidity gap between the core shaft 10 and the wire rod 30, thus suppressing occurrence of local deformation in the vicinity of a joint portion between the core shaft 10 and the wire rod 30, and preventing breakage of the core shaft 10 and the wire rod 30.

Note that the coated part 40 can employ any aspect as long as it has a configuration with less plastic deformability than the wire rod 30 and more plastic deformability than the core shaft 10. For example, the coated part 40 is not limited to a multiple thread coil, and may be a single thread coil formed of one wire, may be a tubular member made of resin, metal, or the like formed tubularly, and may be coated with a hydrophobic resin material, a hydrophilic resin material, or a mixture thereof.

The distal tip 51 is positioned at the distal end of the guide wire 1, and holds integrally the distal end of the wire rod 30 and the distal end of the coil body 20. The distal tip 51 can be formed with any bonding agent in the same manner as the proximal end fixing part 52. The distal tip 51 and the proximal end fixing part 52 can employ the same bonding agent or a different bonding agent. The distal tip 51 may be configured to integrally hold the distal end of the wire rod 30, the distal end of the coil body 20, and the distal end portion of the coated part 40.

The proximal end core shaft 60 is a long-length member formed of stainless steel. The proximal end core shaft 60 includes a joint portion 64, a second increased-diameter portion 61, a second large-diameter portion 62, and a second reduced-diameter portion 63. The joint portion 64 takes an approximately cylindrical shape with a smaller diameter than the diameter of the proximal end of the core shaft 10. The core shaft 10 and the proximal end core shaft 60 are connected via a connection member 70, with the distal end of the joint portion 64 being bonded to the proximal end of the core shaft 10. The connection member 70 is formed of nickel-titanium alloy, also generally referred to as "Ni Ti pipe", and has superior flexibility, kink prevention, and shape memory. The second increased-diameter portion 61 takes an approximately truncated cone shape that has a diameter of the distal end side identical to the diameter of the joint portion 64, and an outer diameter increased from the distal end side to the proximal end side. The second large-diameter portion 62 is a portion having the maximum outer diameter in the proximal end core shaft 60, and takes an approximately cylindrical shape with a constant outer diameter. The second reduced-diameter portion 63 takes an approximately truncated cone shape that has a diameter of the distal end side identical to the diameter of the second large-diameter portion 62, and an outer diameter reduced from the distal end side to the proximal end side.

The proximal end core shaft 60 is used when a technician grips the guide wire 1. The proximal end core shaft 60 is formed of stainless steel, thus has higher rigidity than the core shaft 10 formed of Ni-Ti alloy, and allows improved pushdown and delivery of the guide wire 1. Moreover, stainless steel has superior formability, and thus allows easy production of the proximal end core shaft 60. Examples of the stainless steel available include SUS 302, SUS 304, and SUS 316.

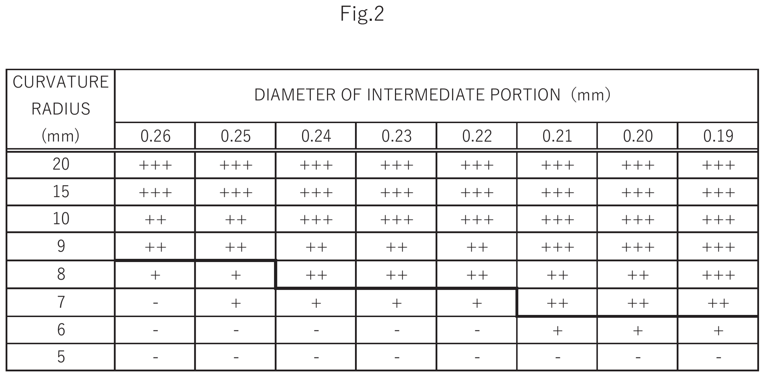

Description will now be provided for effect of the guide wire 1 of the embodiment with reference to Fig. 2-Fig. 5.

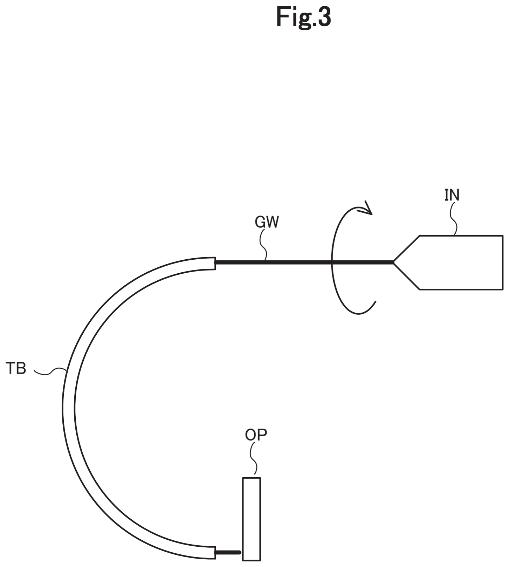

Fig. 2 shows relation of the diameter of an intermediate portion and rotational performance. In Fig. 2, the diameter of the intermediate portion of the guide wire is changed to evaluate rotational performance on the basis of difference between an input angle and an output angle in the test shown in Fig. 3 described later. Fig. 3 is an illustration to illustrate an evaluation test for rotational performance.

In Fig. 2, rotational performance is evaluated for each diameter of the intermediate portions as the curvature radius shown in Fig. 3 is changed to 5, 6, 7, 8, 9, 10, 15, and 20 (mm). In the rotational performance evaluation test shown in Fig. 3, the proximal end side of a guide wire GW is connected to a rotary device IN, and the distal end side is connected to a measuring device OT, with the intermediate portion being inserted in and passed through a tube TB formed with a predetermined curvature. The measuring device OT measures an output angle to an input angle as the proximal end side of the guide wire GW is rotated at a speed of 1.5 rpm in the rotary device IN. In Fig. 2, a difference between an input angle and an output angle of less than 10° is represented by "+++"; a difference between an input angle and an output angle of 10° or more to less than 50° is represented by "++", a difference between an input angle and an output angle of 50° or more to less than 100° is represented by "+", a difference between an input angle and an output angle of 100° or more is represented by "-". The difference between an input angle and an output angle employs a difference as change of an output angle to an input angle is stable.

In Fig. 2, a bold line indicates evaluation between "++" and "+". As shown in Fig. 2, a diameter of the intermediate portion of 0.24 mm or less leads to better rotational performance relative to a diameter of the intermediate portion of 0.25 mm or more. Meanwhile, the human blood vessel has a site with a curvature radius of about 8 mm at a proximal end portion such as a branch of the main trunk and the circumflex of the left coronary artery. In other words, a guide wire is possibly used in a site having a curvature radius of about 8 mm. It is thus preferable to use a guide wire with rotational performance evaluated as "++" or "+++" at a curvature radius of 8 mm. That is, in view of rotational performance, the diameter of the intermediate portion is preferably less than 0.25 mm. By contrast, a core shaft formed of nickel-titanium (Ni-Ti) alloy has less flexural rigidity than a core shaft formed of stainless alloy, and thus preferably has a larger diameter of the core shaft in view of pushdown and delivery. Accordingly, for balancing pushdown and delivery with rotational performance, the diameter of the intermediate portion is preferably 0.20 mm or more to 0.24 mm or less, which has rotational performance evaluated as "++" at a curvature radius of 8 mm. More preferable is 0.22 mm or more to 0.24 mm or less. The embodiment employs 0.24 mm, the largest diameter in the range described above.

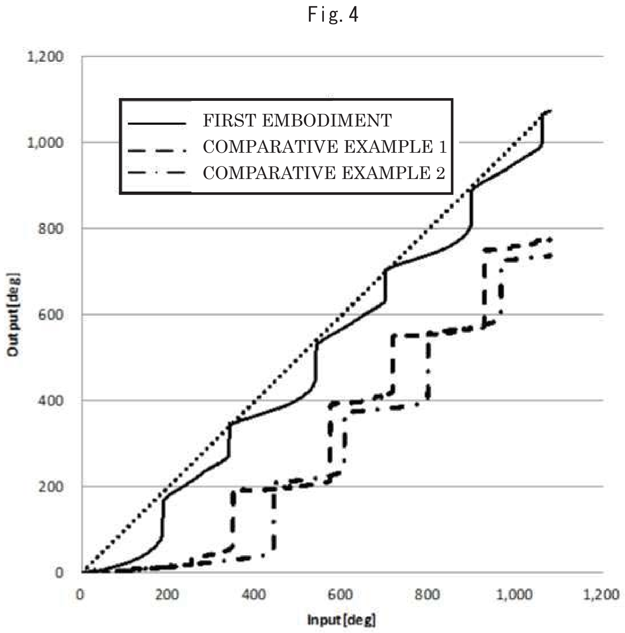

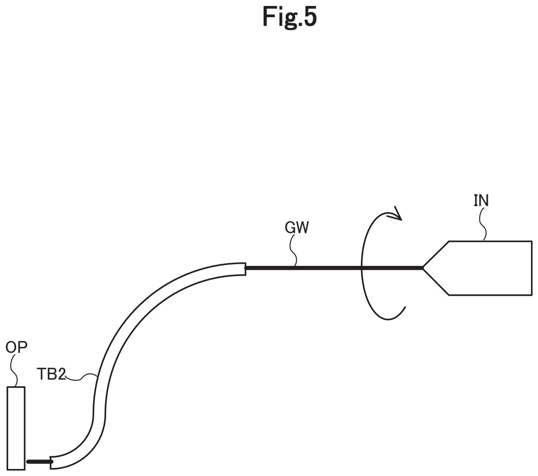

Fig. 4 shows rotational performance of the guide wire 1 of the embodiment. Fig. 4 illustrates comparison to the comparative examples having difference in the diameter of the intermediate portion from the guide wire 1 of the embodiment. Fig. 5 is an illustration to illustrate the evaluation test for rotational performance shown in Fig. 4.

The rotational performance evaluation test shown in Fig. 5 is a test similar to the test shown in Fig. 3, but a tube TB2 is different from the tube TB shown in Fig. 3 and has a two-step curvature. In the tube TB2, the curvature close to the rotary device IN has a curvature radius of 70 mm, and the curvature close to the measuring device OT has a curvature radius of 3 mm. Fig. 4 shows an output angle to an input angle as the proximal end side of the guide wire GW is rotated at a speed of 1.5 rpm in the rotary device IN. Fig. 4 depicts the guide wire 1 of the embodiment by a solid line, Comparative Example 1 by a dashed line, and Comparative Example 2 by a dash-dot line. Comparative Examples 1 and 2 are third-party products each of which has an intermediate portion with a diameter of 0.25 mm in a guide wire. Fig. 4 indicates ideal behavior with no delay of torque response, by a dotted line. As shown in Fig. 5, the guide wire 1 of the embodiment exhibits slight delay of torque response, but displays nearly ideal behavior. In other words, the guide wire 1 of the embodiment enabled suppressing delay of torque response relative to the guide wires of the comparative examples.

As described above, the guide wire 1 of the embodiment provides the intermediate portion 15 of the core shaft 10 with a relatively thin diameter, 0.24 mm, and thus allows suppression of delay of torque response. In other words, a guide wire with good rotational performance can be provided.

Moreover, the guide wire 1 of the embodiment includes the coil body 20 with an outer diameter of 0.36 mm or less that covers a part of the intermediate portion 15 and the tapered portion 12 of the core shaft 10, and thus enables ensuring a proper size of the guide wire 1 even in a thin part of the core shaft 10.

Furthermore, in the guide wire 1 of the embodiment, the coil body 20 has a constant wire diameter (mean diameter of the wire 21). For example, when an intermediate portion having a larger diameter than the intermediate portion 15 is used instead of the intermediate portion 15 of the guide wire 1 of the embodiment, a coil body is set to have a smaller wire diameter in a part covering the intermediate portion than the wire diameter in a part covering the tapered portion 12 in order to keep a constant outer diameter of the coil body. Use of a guide wire employing such coil body may result in riding on, collapse, or the like of the coil body in a part of the coil body having a smaller wire diameter, during use of the guide wire. By contrast, the guide wire 1 of the embodiment provides a relatively thick, constant wire shape of the coil body 20 by setting the diameter of the intermediate portion 15 to 0.24 mm (to be thin), and thus allows suppressing occurrence of riding on, collapse, or the like of the coil body 20 during use of the guide wire 1.

Additionally, the guide wire 1 of the embodiment employs a core shaft made of nickel-titanium alloy in the vicinity of the distal end side. Nickel-titanium alloy is a superelastic metal and thus can provide a guide wire with restorability. Particularly, since nickel-titanium alloy has superior restorability, durability, and corrosion resistance among superelastic metals, the guide wire 1 of the embodiment enables improving restorability, durability, and corrosion resistance of the core shaft 10.

Moreover, the guide wire 1 of the embodiment has the coil body 20 wound around from a predetermined position of the intermediate portion 15 of the core shaft 10 to the distal end of the guide wire 1, and thus allows increased flexural rigidity of the intermediate portion 15 and the tapered portion 12, which are relatively thin parts in the guide wire 1.

Furthermore, the guide wire 1 of the embodiment includes the proximal end core shaft 60 formed of stainless steel causing higher rigidity of the proximal end core shaft 60 than that of the core shaft 10, and thus allows improved pushdown and delivery. Consequently, it is possible to provide the guide wire 1 having high pushdown and delivery, and superior rotational performance.

As described above, the core shaft 10 is formed of a superelastic material, and the wire rod 30 is formed of a material having more plastic deformability than that of the core shaft 10. The coated part 40 is configured to have less plastic deformability than the wire rod 30 and more plastic deformability than the core shaft 10. The coated part 40 enables reducing rigidity gap between the core shaft 10 and the wire rod 30 having different rigidity, and thus allows shaping onto a joint portion between the core shaft 10 and the wire rod 30 to be easier relative to a configuration without the coated part 40. Moreover, reducing rigidity gap between the core shaft 10 and the wire rod 30 in the coated part 40 protects a locally deformable part generated in the vicinity of the joint portion between the core shaft 10 and the wire rod 30, and allows suppressing breakage of the core shaft 10 and the wire rod 30. Consequently, it is possible to improve durability of the guide wire 1.

Additionally, in the guide wire 1 of the embodiment, the distal end portion of the coated part 40 is located forward relative to the proximal end portion of the wire rod 30 and coats a half or more of the wire rod 30 (Fig. 1). In other words, in the guide wire 1 of the embodiment, the coated part 40 is positioned up to the vicinity of the distal tip 51. In this manner, protection is made for a major part of the wire rod 30, which is formed of a material having high plastic deformability, thereby allowing suppressing breakage of the wire rod 30 in association with shaping and use, and providing more improved durability of the guide wire 1.

### <Second Embodiment>

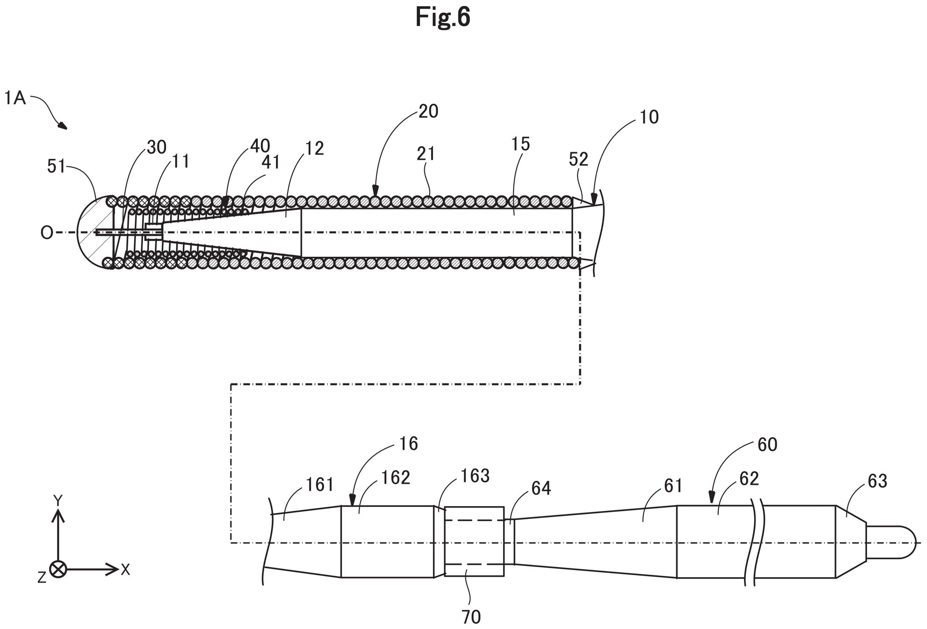

Fig. 6 is a partial sectional view showing a configuration of a guide wire 1A of a second embodiment. The guide wire 1A in the second embodiment includes the proximal end fixing part 52 on the first increased-diameter portion 161 of the proximal end portion 16. In other words, the proximal end portion of the coil body 20 is not fixed to the intermediate portion 15 of the core shaft 10, and is fixed to the proximal end portion 16 of the core shaft 10.

The guide wire 1A of the embodiment has a coil body 20 wound around from the proximal end side of the intermediate portion 15 of the core shaft 10 to the distal end of the guide wire 1A, and thus allows increasing flexural rigidity of the whole of the intermediate portion 15 and the tapered portion 12, which are relatively thin parts in the guide wire 1A.

### <Third Embodiment>

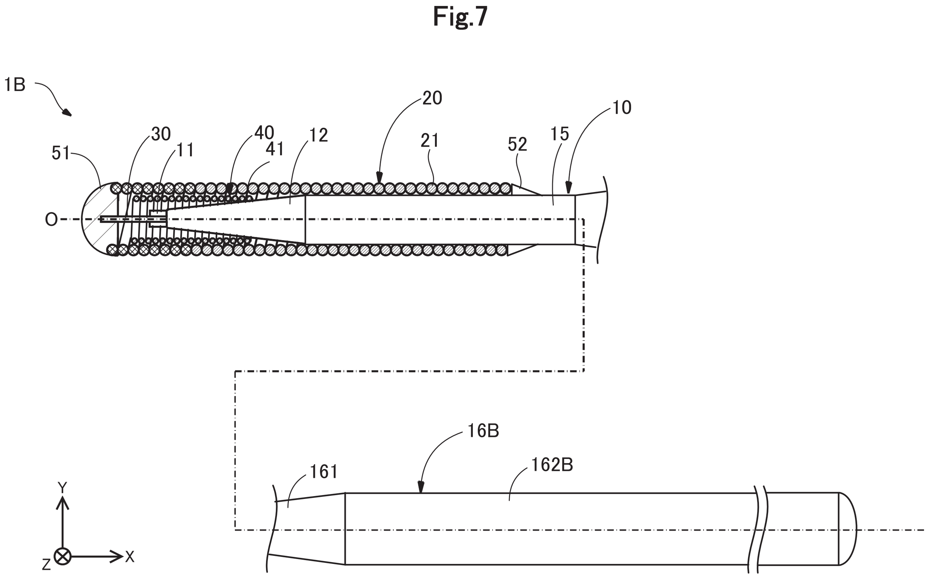

Fig. 7 is a partial sectional view showing a configuration of a guide wire 1B of a third embodiment. The guide wire 1B of the third embodiment does not include the proximal end core shaft 60. In other words, a proximal end portion 16B formed of nickel-titanium alloy is positioned at the proximal end of the guide wire 1B of the embodiment. The proximal end portion 16B in the guide wire 1B of the embodiment has a first large-diameter portion 162B with a longer length relative to the proximal end portion 16 in the guide wire 1 of the first embodiment, and does not include the first reduced-diameter portion 163.

The guide wire 1B of the embodiment does not include the proximal end core shaft 60 made of stainless steel, but has placement of the first large-diameter portion 162B, which has the largest diameter in the core shaft 10, at the proximal end of the guide wire 1B, and thus enables ensuring pushdown and delivery. In other words, the embodiment can also provide the guide wire 1B, which has good pushdown and delivery, and rotational performance.

### <Fourth Embodiment>

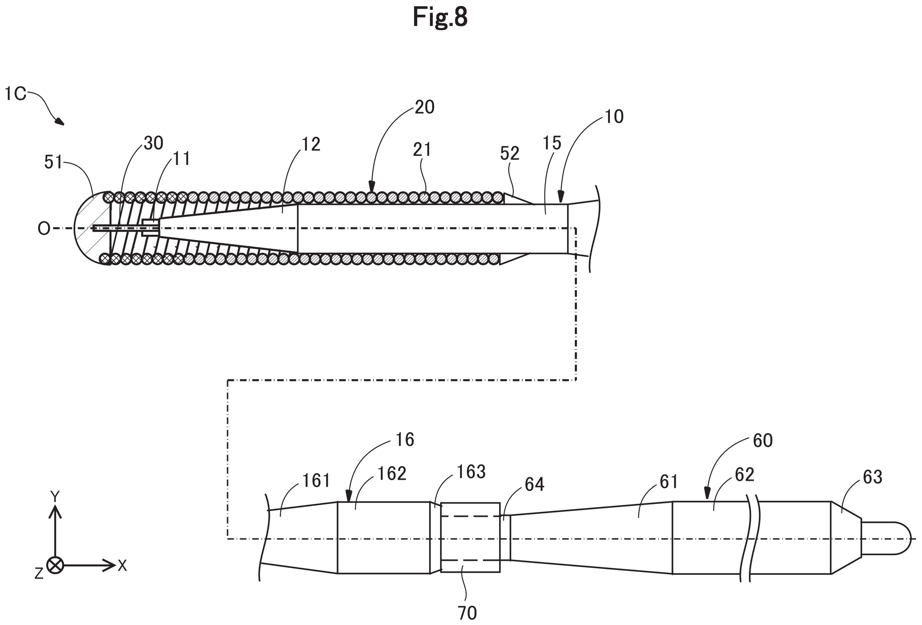

Fig. 8 is a partial sectional view showing a configuration of a guide wire 1C of a fourth embodiment. The guide wire 1C of the fourth embodiment does not include the coated part 40. Nevertheless, this includes the coil body 20, and thus ensures flexural rigidity of the vicinity of the distal end of the guide wire 1C. Even the guide wire 1C of the fourth embodiment can provide an effect similar to the first embodiment.

### <Fifth Embodiment>

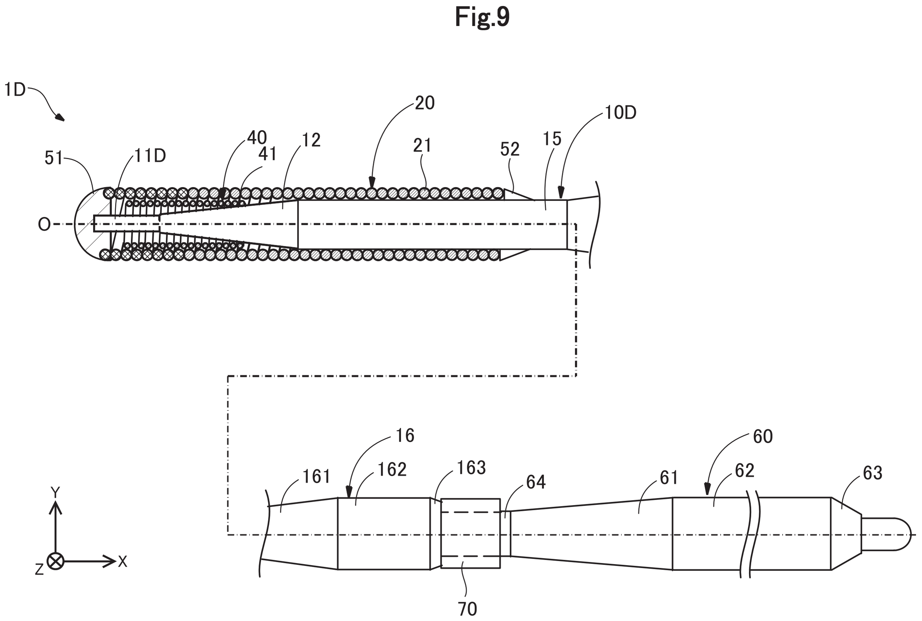

Fig. 9 is a partial sectional view showing a configuration of a guide wire 1D of a fifth embodiment. The guide wire 1D of the fifth embodiment does not include the wire rod 30. In other words, as illustrated, a small-diameter portion 11D of the core shaft 10D extends up to the distal tip 51. In addition, the distal tip 51 integrally holds the distal end of the core shaft 10D and the distal end of the coil body 20. Even with this, the intermediate portion 15 of the core shaft 10D has a relatively thin diameter, 0.24 mm, and it is thus possible to suppress delay of torque response.

### <Variants of the Embodiment>

The disclosed embodiments are not limited to the embodiments described above, and can be performed in various aspects in the range without departing from the spirit, and for example, the following variants are available.
- The guide wire of each of the embodiments described above have been described as a medical appliance used in insertion of a catheter into a blood vessel, but can also be configured as a guide wire to be inserted into each of the organs in the human body such as the lymph gland system, biliary system, urinary system, respiratory tract system, digestive organ system, secretory gland, or genital organs.
- In each of the embodiments described above, the intermediate portion 15 formed in an approximately cylindrical shape with a constant diameter has been exemplified, but the shape of the intermediate portion is not limited to the embodiments described above. For example, an approximately cylindrical shape may be formed with changing the diameter of the intermediate portion continuously or intermittently. When the diameter of the intermediate portion is changed in this manner, the mean diameter may be 0.22 mm or more to 0.24 mm or less, or the diameter may be changed in the range of 0.22 mm or more to 0.24 mm or less.
- In the embodiments described above, the core shaft 10 formed of nickel-titanium (Ni-Ti) alloy has been exemplified, but the material forming the core shaft 10 is not limited to the embodiments described above. Various superelastic metals can be used such as Ni-Ti-based alloy, which is alloy of Ni-Ti and another metal such as Cu, and Cu-based alloy such as Cu-Zn-Al alloy.
- The configuration of the core shaft is not limited to the embodiments described above. For example, the core shaft 10 of the embodiments described above may not include the small-diameter portion 11. When the core shaft does not include a small-diameter portion, the wire rod 30 can be bonded to a tapered portion of the core shaft. Moreover, in each of the embodiments, the core shaft may be configured of a plurality of bound core shaft members. In this case, each of the core shaft members may be formed of the same material, or may be formed of different superelastic metals.
- In the embodiments described above, the proximal end core shaft 60 formed of stainless steel has been exemplified, but the proximal end core shaft 60 can be formed of various materials having higher rigidity than that of the core shaft 10. For example, a high rigidity material can be used such as chromium-molybdenum steel, nickel-chromium-molybdenum steel, Inconel (Inconel as a trademark), Incoloy (Incoloy as a trademark).
- The cross-sectional shape of each of the parts bonding the small-diameter portion 11 of the core shaft 10 to the wire rod 30 is not limited to the embodiments described above. For example, various shapes can be employed such as an approximately circular shape, a polygonal shape, a shape having a groove in an approximate circle, ellipse, or the like.
- In the embodiments described above, the coil body 20 having a constant wire diameter has been exemplified, but the wire diameter of the coil body 20 may not be constant. For example, the wire diameter of a part of the coil body 20 covering the tapered portion 12 of the core shaft 10, the small-diameter portion 11, and the wire rod 30 may be larger than the wire diameter of a part of the coil body 20 covering the intermediate portion 15.
- The configuration of the coil body is not limited to the embodiment described above. For example, the coil body may be configured to be densely wound with no space between adjacent wires, or may be sparsely wound with a space between adjacent wires, or may have a configuration with a mixture of dense winding and sparse winding. In addition, the coil body may include a resin layer coated with, e.g., a hydrophobic resin material, a hydrophilic resin material, or a mixture thereof. For example, the cross-sectional shape of the wire of the coil body may not be approximately circular.
- In the embodiments described above, an example has been shown where the coil body 20 is formed of platinum nickel (Pt-Ni) alloy in the vicinity of the distal end, and of stainless alloy in a portion closer to the proximal end, but the coil body may be wholly formed of the same material. Additionally, formation may be made by, e.g., using three or more different materials and changing a material along an axis direction.

The aspects have been described on the basis of embodiments and variants so far, but the embodiments of the aspect described above are provided to facilitate understanding the aspects and not to limit the aspects. The aspects can be altered or modified without departing from the spirit and scope, as well as encompass the equivalents thereof. In addition, the technical features can be appropriately deleted as long as it has not been described as an essential feature herein.

### DESCRIPTION OF REFERENCE NUMERALS

1, 1A, 1B, 1C, 1D: guide wire
10, 10D: core shaft
11, 11D: small-diameter portion
12: tapered portion
15: intermediate portion
16, 16B: proximal end portion
20: coil body
21: wire
30: wire rod
40: coated part
41: wire
51: distal tip
52: proximal end fixing part
60: proximal end core shaft
61: second increased-diameter portion
62: second large-diameter portion
63: second reduced-diameter portion
64: joint portion
70: connection member
161: first increased-diameter portion
162, 162B: first large-diameter portion
163: first reduced-diameter portion
GW: guide wire
IN: rotary device
O: axis
OT: measuring device

## Claims

1. A guide wire (1, 1A, 1B, 1C 1D), comprising:
a core shaft (10, 10D) formed of superelastic metal, wherein the core shaft (10, 10D) has
a tapered portion (12) having a diameter reducing from its own proximal end side toward the distal end side, an intermediate portion (15) having an approximately cylindrical shape adjacent to the proximal end side of the tapered portion (12),
and a proximal end portion (16, 16B) adjacent to the proximal end side of the intermediate portion (15) and having a larger diameter than the intermediate portion (15);
a coil body (20) covering at least a part of the intermediate portion (15) and the tapered portion (12) of the core shaft (10, 10D), the coil body (20) having an outer diameter of 0.36 mm or less; and
a distal tip (51) disposed at the distal end of the coil body (20) and forming the distal end of the guide wire (1, 1A, 1B, 1C 1D),
**characterized in that**
the guide wire (1, 1A, 1B, 1C, 1D) comprises a coated part (40) covering at least a distal end side of the tapered portion (12) with a proximal end side of the coated part (40) being bonded to the tapered portion (12) of the core shaft (10, 10D) and being a tubular member that is configured to have more plastic deformability than the core shaft (10, 10D); and
**in that** the diameter of the intermediate portion (15) of the core shaft (10, 10D) is 0.22 mm or more to 0.24 mm or less.

2. The guide wire according to claim 1, wherein the coil body (20) has constant wire diameter.

3. The guide wire according to claim 1 or claim 2, wherein the core shaft (10, 10D) is formed of nickel-titanium (Ni-Ti) alloy.

4. The guide wire according to any one of claim 1 to claim 3, wherein the proximal end portion of the coil body (20) is fixed to the intermediate portion (15) of the core shaft (10, 10D).

5. The guide wire according to any one of claim 1 to claim 4, further comprising a proximal end core shaft (60) connected to the proximal end side of the core shaft (10, 10D) and formed of a material with higher rigidity than that of the core shaft (10, 10D).

6. The guide wire according to claim 5, wherein the proximal end core shaft (60) is formed of stainless steel.

7. The guide wire according to any one of claims 1 to 6, further including a wire rod (30) connected to the distal end of the core shaft (109, 10D) and formed of a material having more plastic deformability than that of the core shaft (10, 10D), wherein the distal tip (51) is connected between the distal end of the coil body (20) and the distal end of the wire rod (30).

8. The guide wire according to claim 7, wherein the coated part (40) also covers the proximal end of the wire rod (30) and is configured to have less plastic deformability than the wire rod (30).
